(19) Europäisches Patentamt
European Patent Office
Office européen des brevets

(11) Veröffentlichungsnummer: **0 318 855**
**A2**

(12) # EUROPÄISCHE PATENTANMELDUNG

(21) Anmeldenummer: **88119652.1**

(22) Anmeldetag: **25.11.88**

(51) Int. Cl.⁴: **C07D 311/92 , C07D 405/12 , C07D 413/12 , C07D 405/14 , A61K 31/35**

Patentansprüche für folgende Vertragsstaaten: ES + GR.

(30) Priorität: **01.12.87 DE 3740624**

(43) Veröffentlichungstag der Anmeldung: **07.06.89 Patentblatt 89/23**

(84) Benannte Vertragsstaaten: **AT BE CH DE ES FR GB GR IT LI LU NL SE**

(71) Anmelder: **HOECHST AKTIENGESELLSCHAFT**
**Postfach 80 03 20**
**D-6230 Frankfurt am Main 80(DE)**

(72) Erfinder: **Lal, Bansi, Dr.**
**30 Advani Apartments**
**Mulund (West) Bombay 400 080(IN)**
Erfinder: **Gangopadhya, Ashok Kumar**
**B-16 Hoechst Staff Quarters Darga Road**
**Mulund (West) Bombay 400 082(IN)**
Erfinder: **Dohadwalla, Alihussein Nomanbhai, Dr.**
**Noman Mansion, 139 C**
**Cumballa Hill Bombay 400 036(IN)**
Erfinder: **Rajgopalan, Ramanujam, Dr.**
**203, B Wing Arthi Sarojini Naidu Road**
**Mulund (West) Bombay 400 080(IN)**
Erfinder: **Rupp, Richard Helmut, Dr.**
**Roederweg 16a**
**D-6240 Königstein/Taunus(DE)**

(54) **Neue Labdan-Derivate, ein Verfahren zu ihrer Herstellung und ihre Verwendung als Arzneimittel.**

(57) Die vorliegende Erfindung betrifft polyoxygenierte Labdanderivate der Formel

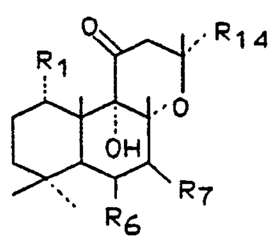

I

ein Verfahren zu ihrer Herstellung und die Anwendung der Substanzen als pharmazeutische Mittel, insbesondere als Medikamente mit Wirkung gegen erhöhten Augeninnendruck und erhöhten Blutdruck.

EP 0 318 855 A2

## Neue Labdan-Derivate, ein Verfahren zu ihrer Herstellung und ihre Verwendung als Arzneimittel

Die vorliegende Erfindung betrifft neue substituierte Acylderivate von polyoxylierten Hydroxyacyloxy-, Aminoacyloxy- und Thioacyloxy-Labdanen und ihre pharmakologisch verwendbaren Salze, ein Verfahren zu ihrer Herstellung und ihre Verwendung als Arzneimittel.

Polyoxylierte Labdane und ihre Derivate sind bereits beschrieben worden, z.B. in: Deutsche Offenlegungsschriften Nr. 2 557 784, 2 640 275 und 2 654 796; Tetrahedron Letters Nr. 19, Seiten 1669-1672 (1977); J. Chem. Soc., Perkin Trans. 1, 767 (1982), sowie in den europäischen Patentanmeldungen EP-A-0 217 372, EP-A-0 191 166 und EP-A-0 193 132.

Infolge der pharmakologischen Eigenschaften der polyoxylierten Labdane und ihrer Derivate sind sie geeignet zur Behandlung von Herz- und Kreislauferkrankungen, hohem Blutdruck, Glaukom, Allergien und Bronchialasthma. Sie wirken ferner als Immunomodulatoren und besitzen Adenylatcyclase-stimulierende Wirkung.

Die polyoxylierten Labdane gemäß der Erfindung wird weder in den als Stand der Technik genannten Publikationen beschrieben noch sind sie durch diese nahegelegt. Verbindungen des Standes der Technik, die in einigen Fällen mit den Verbindungen gemäß der Erfindung strukturell verwandt sind, sind die Derivate, die eine 6- oder 7-Aminoacyloxygruppe oder Hydroxyacyloxygruppe aufweisen.

Der wesentliche Unterschied zwischen den Verbindungen der Erfindung und jenen des Standes der Technik ist der, daß die Verbindungen gemäß der Erfindung Acylderivate dieser 6- bzw. 7-Aminoacyloxy- oder Hydroxyacyloxy-Funktion sind. Überraschenderweise ändert dieser strukturelle Wechsel das pharmakologische Profil der Verbindungen dahingehend, daß sie sich besser zur Behandlung von Herzinsuffizienzkrankheiten, wie kongestive Kardiomyopathie und verwandte Indikationen, und Hypertonie eignen.

Die vorliegende Erfindung betrifft daher neue Derivate polyoxylierter Labdane der Formel I

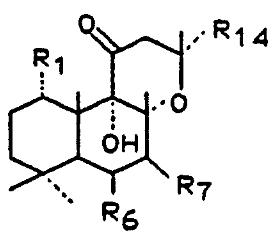

I

worin bedeuten

$R_1$ OH, O-Alkyl oder einen Rest der Formel II

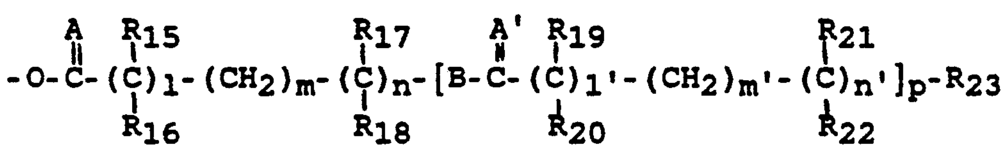

II

worin A und A′ für Sauerstoff oder Schwefel, B für $-CH_2-$, Sauerstoff, Schwefel oder -NH-, $R_{15}$-$R_{23}$ für Wasserstoff, Alkyl, Aryl, Aralkyl, Hydroxy, Alkoxy, Thiol, Halogen oder eine Gruppe der Formel $NR_{24}R_{25}$ stehen, worin $R_{24}$ und $R_{25}$, falls sie gleich sind, Wasserstoff, Alkyl, substituiertes Alkyl, Aryl oder Aralkyl bedeuten, oder falls $R_{24}$ für Wasserstoff steht, $R_{25}$ Alkyl, substituiertes Alkyl, Cycloalkyl, Aralkyl, Aryl, einen Heterocyclus, Amino, Dialkylamino, Alkylamino, Arylamino, Aralkylamino, Hydroxy, Thiol, Acyloxy, Acyl, Carbamoyl, Carboxyalkyl, Carbalkoxyalkyl, Dialkylaminoalkyl bedeutet, oder falls $R_{24}$ für Alkyl steht, $R_{25}$ substituiertes Alkyl, Cycloalkyl, Aryl, Aralkyl oder Dialkylaminoalkyl bedeutet, oder $R_{24}$ und $R_{25}$ zusammen mit dem Stickstoffatom, an das sie gebunden sind, für einen Heterocyclus stehen, der ein oder mehrere Heteroatome aufweisen und gegebenenfalls ein- oder mehrfach mit Alkyl, Aryl, Hydroxyalkyl, Halogen, Hydroxy, Alkoxy oder anderen heterocyclischen Gruppen substituiert sein kann, mit der Maßgabe, daß der Rest mindestens drei der Substituenten $R_{15}$-$R_{23}$ enthält, wobei mindestens einer der drei Substituenten ein Heteroatom der Gruppe N, O oder S aufweist,

und

l, m, n, l′, m′, n′ jeweils 0 oder eine ganze Zahl von 1 bis 10, und p eine ganze Zahl von 1 bis 10 bedeuten, $R_6$ OH, O-Alkyl oder eine Gruppe der Formel II

$$-O-\overset{A}{\overset{\|}{C}}-(\overset{R_{15}}{\underset{R_{16}}{C}})_{l}-(CH_2)_{m}-(\overset{R_{17}}{\underset{R_{18}}{C}})_{n}-[B-\overset{A'}{\overset{\|}{C}}-(\overset{R_{19}}{\underset{R_{20}}{C}})_{l'}-(CH_2)_{m'}-(\overset{R_{21}}{\underset{R_{22}}{C}})_{n'}]_{p}-R_{23} \qquad II$$

worin A, A′, l, m, n, l′, m′, n′, p und $R_{15}$-$R_{23}$ die gleiche Bedeutung wie oben angegeben haben, $R_7$ OH, O-Alkyl oder eine Gruppe der Formel II

$$-O-\overset{A}{\overset{\|}{C}}-(\overset{R_{15}}{\underset{R_{16}}{C}})_{l}-(CH_2)_{m}-(\overset{R_{17}}{\underset{R_{18}}{C}})_{n}-[B-\overset{A'}{\overset{\|}{C}}-(\overset{R_{19}}{\underset{R_{20}}{C}})_{l'}-(CH_2)_{m'}-(\overset{R_{21}}{\underset{R_{22}}{C}})_{n'}]_{p}-R_{23} \qquad II$$

worin A, A′, l, m, n, l′, m′, n′, p und $R_{15}$-$R_{23}$ die gleiche Bedeutung wie oben angegeben haben, $R_{14}$ Vinyl, Ethyl, Cyclopropyl, $CHOHCH_2OH$, $CH_2OH$ oder $-\overset{Z}{\overset{|}{C}}=CH_2$, worin Z für Halogen, wie Chlor, Brom oder Fluor steht, mit der Maßgabe, daß

a) $R_1$, $R_6$ und $R_7$ nicht gleichzeitig OH-Gruppen sind,

b) falls $R_7$ $O-\overset{O}{\overset{\|}{C}}-CH_3$ ist, $R_1$ und $R_6$ nicht OH sind,

sowie deren pharmakologisch unbedenklichen Säureadditionssalze.

Eine bevorzugte Gruppe von Verbindungen der vorliegenden Erfindung sind Verbindungen der Formel I, worin

$R_1$ die OH-Gruppe bedeutet,

eines der Substituenten $R_6$ und $R_7$ OH, O-Alkyl oder O-Acyl bedeutet und der andere eine Untergruppe des oben beschriebenen Restes, dargestellt durch die Formel III

$$-O-\overset{O}{\overset{\|}{C}}-(\overset{R_{15}}{\underset{R_{16}}{C}})_{l}-[B-\overset{O}{\overset{\|}{C}}-(CH_2)_{m'}]_{p}-R_{23} \qquad III$$

darstellt,

worin $R_{15}$, $R_{16}$, $R_{23}$, B, l und m′ die gleiche Bedeutung wie oben angegeben haben, p = 1 ist und $R_{14}$ die oben genannte Bedeutung hat, sowie deren pharmakologisch unbedenklichen Säureadditionssalze.

Besonders bevorzugt sind Verbindungen der Formel I, worin $R_1$ die OH-Gruppe, $R_{14}$ die Vinylgruppe, einer der Substituenten $R_6$ und $R_7$ OH, O-$C_1$-$C_4$-Alkyl oder O-$C_1$-$C_4$-Alkanoyl bedeuten, und der andere den Rest der Formel III darstellt, worin $R_{15}$ und $R_{16}$ jeweils Wasserstoff, B ein Sauerstoffatom, $R_{23}$ den Rest

$$-N\begin{matrix} / R_{24} \\ \backslash R_{25} \end{matrix} ,$$

l und p die Zahl 1 und m′ eine ganze Zahl von 1 bis 4 bedeuten, sowie deren pharmakologisch unbedenklichen Säureadditionssalze.

Geeignete Beispiele für die Definition Alkyl der Substituenten $R_{15}$-$R_{25}$ sind geradkettige oder verzweigte Alkylreste mit bis zu 6 und vorzugsweise bis zu 4 Kohlenstoffatomen, z.B. Methyl, Ethyl, Isopropyl, t-Butyl, n-Butyl.

Geeignete Beispiele für substituierte Alkylgruppen in der Bedeutung für $R_{24}$ und $R_{25}$ sind Hydroxy-$C_1$-$C_6$-alkyl, wie Hydroxyethyl, Carboxy-$C_1$-$C_6$-alkyl, wie Carboxyethyl, Carb-$C_1$-$C_6$-alkoxyalkyl, wie Carbethoxyethyl.

Geeignete Beispiele für Cycloalkylgruppen in der Bedeutung für $R_{24}$ und $R_{25}$ sind $C_3$-$C_7$-Cycloalkylgruppen, insbesondere Cyclopentyl oder Cyclohexyl.

Geeignete Beispiele für Aralkylgruppen in der Bedeutung für $R_{24}$ und $R_{25}$ sind Phenylalkylgruppen, insbesondere Phenyl-$C_1$-$C_3$-alkyl, z.B. die Benzylgruppe, worin die Phenylgruppe durch einen oder mehrere Substituenten, wie Halogen, $C_1$-$C_3$-Alkyl, $C_1$-$C_3$-Alkoxy, Nitro oder Trifluormethyl, substituiert sein kann.

Ein geeignetes Beispiel für Arylgruppen in der Bedeutung für $R_{24}$ und $R_{25}$ ist die Phenylgruppe, die durch einen oder mehrere Substituenten, wie Halogen, $C_1$-$C_3$-Alkyl, $C_1$-$C_3$-Alkoxy, Nitro oder Trifluormethyl, substituiert sein kann.

Geeignete Beispiele für Acylgruppen in der Bedeutung für $R_1$, $R_7$ und $R_{25}$ sind $C_1$-$C_6$-Alkanoyl, $C_2$-$C_6$-Alkenoyl, $C_3$-$C_6$-Alkinoyl, Aroyl, Aryl-$C_1$-$C_6$-alkanoyl oder eine Heteroaroylgruppe mit bis zu 10 Kohlenstoffatomen, worin ein oder mehrere C-Atome durch Sauerstoff, Stickstoff und/oder Schwefel ersetzt sein können.

Beispiele für solche Alkanoylgruppen sind Formyl, Acetyl, Propionyl, Butyryl, Isobutyryl, Valeryl, Palmityl, Bromisobutyryl, bevorzugt Formyl, Acetyl oder Propionyl. Die Alkanoylgruppen können eine oder mehrere Doppelbindungen enthalten, z.B. Acryloyl, Stearoyl oder Oleoyl. Die Alkanoylgruppen können auch eine oder mehrere Dreifachbindungen und zusätzlich eine oder mehrere Doppelbindungen enthalten. Ein Beispiel für Alkinoylgruppen ist Propiolyl. Aroylgruppen werden durch Benzoyl repräsentiert, in der die Phenylgruppe gegebenenfalls durch einen odere mehrere Substituenten substituiert sein kann, wie $C_1$-$C_3$-Alkyl, $C_1$-$C_3$-Alkoxy, Halogen, Nitro oder Trifluormethyl. Beispiele für Aralkanoyl- oder Heteroaroylgruppen sind Phenylacetyl bzw. Pyridin-3-carbonyl.

Unter Dialkylaminoalkylgruppen sind solche zu verstehen, in denen die Alkylgruppen jeweils 1 bis 6 C-Atome enthalten, z.B. Diethylaminoethyl. Falls $R_{24}$ und $R_{25}$ zusammen mit dem Stickstoffatom, an das sie gebunden sind, einen Heterocyclus darstellen, sind Piperidin, Pyrrolidin, Morpholin, Piperazin, Thiomorpholin, Imidazol oder Theophyllin, die gegebenenfalls ein- oder mehrfach durch $C_1$-$C_4$-Alkyl, $C_1$-$C_4$-Alkoxy, Aryl, Aryl-$C_1$-$C_4$-Alkyl, Hydroxy, Amino oder substituiertes $C_1$-$C_4$-Alkyl, substituiert sein können, bevorzugt.

Geeignete Beispiele für Salze der Verbindungen der Erfindung mit anorganischen oder organischen Säuren sind Hydrochlorid, Hydrobromid, Sulfat, Phosphat, Acetat, Oxalat, Tartrat, Citrat, Maleat oder Fumarat.

In den hier angegebenen Formeln werden die verschiedenen Substituenten dargestellt als mit dem Labdankern verbunden in einer von zwei Bezeichnungsweisen: Eine ausgezogene Linie (__), die einen Substituenten in $\beta$-Stellung (d.h. oberhalb der Molekülebene) angibt und eine gestrichelte Linie (-----), die einen Substituenten in $\alpha$-Stellung (d.h. unterhalb der Molekülebene) angibt. Die Formeln sind alle so bezeichnet, daß sie die Verbindungen in ihrer absoluten stereochemischen Konfiguration angeben. Da die Ausgangsverbindungen, die einen Labdankern aufweisen, natürlich vorkommen oder aus natürlich vorkommenden Verbindungen abgeleitet sind, haben sie und auch die Endprodukte einen Labdankern, der in der einzigen hier angegebenen absoluten Konfiguration existiert. Das Verfahren der vorliegenden Erfindung ist jedoch gleichfalls zur Anwendung auf die Synthese von Labdanen der racemischen Serie bestimmt.

Zusätzlich zu den optischen Zentren des Labdankerns können auch die Substituenten daran chirale Zentren aufweisen, die zu den optischen Eigenschaften der Verbindungen der vorliegenden Erfindung beitragen und ein Mittel zur Auftrennung durch konventionelle Methoden bieten, z.B. durch die Verwendung optisch aktiver Säuren.

Eine Wellenline (~), die eine Gruppe mit einem chiralen Zentrum verbindet, zeigt an, daß die Stereochemie des Zentrums unbekannt ist, d.h. die Gruppe kann in irgendeiner der möglichen Orientierungen existieren. Die vorliegende Erfindung umfaßt alle optischen Isomeren und racemischen Formen der Verbindungen der vorliegenden Erfindung, wenn solche Verbindungen chirale Zentren zusätzlich zu jenen des Labdankerns aufweisen.

Einige der bevorzugten neuen Verbindungen der Erfindung der Formel I, worin $R_1$ = OH; $R_{14}$ = CH-$CH_2$; und $R_6$ und $R_7$ entweder OH,

O

O C $CH_3$ oder die bevorzugte oben beschriebene Teilgruppe sind, werden in Tabelle I mit der Formel Ia und in Tabelle II mit der Formel Ib dargestellt.

**Tabelle I**

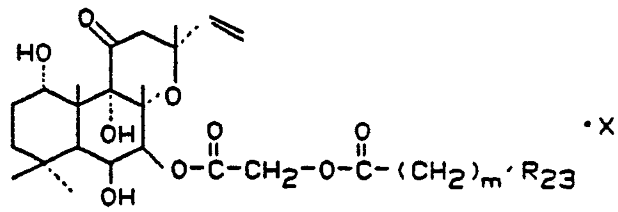

Ia

| m' | $R_{23}$ | X | Schmp. (°C) |
|---|---|---|---|
| 1 | $NH_2$ | $HCl \cdot H_2O$ | 167-172 |
| 1 | NHBoC | - | Schaum |
| 1 | $NHCPh_3$ | - | Schaum |
| 1 | 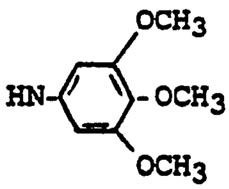 | - | 108-109 |
| 1 | $N[CH(CH_3)_2]_2$ | HCl | 148-150 |
| 1 | $N[CH_2-CH=CH_2]_2$ | HCl | 135-136 |
| 1 | $N(C_2H_5)_2$ | HCl | 140-142 |
| 1 | 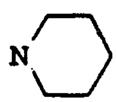 | - | 103-104 |
| 1 | 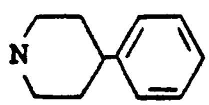 | - | 99-101 |
| 1 | 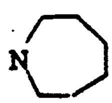 | $HCl \cdot 1,5H_2O$ | 146-148 |
| 1 | 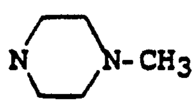 | - | 95-97 |
| 1 | 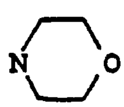 | - | 82 |
| 1 | 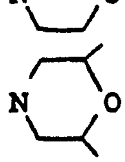 | HCl | 158-160 |
| 1 | 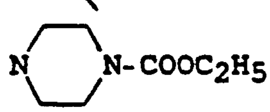 | HCl | 157-158 |
| 2 | $N(CH_3)_2$ | $HCl \cdot 2H_2O$ | 214-215 |

**Tabelle II**

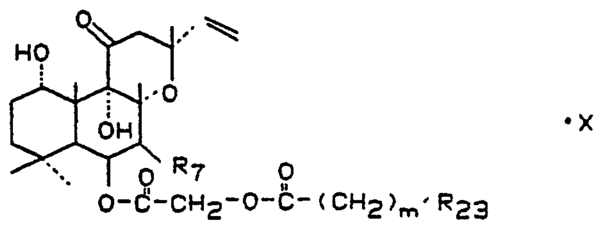

Ib

| $R_7$ | m' | $R_{23}$ | X | Schmp. (°C) |
|---|---|---|---|---|
| OH | 1 | $NH_2$ | $HCl \cdot H_2O$ | 165-167 |
| OAc | 1 | $NH_2$ | HCl | 181-183 |
| OAc | 1 | $NHCPh_3$ | - | Schaum |
| OAc | 1 | $N(CH_3)_2$ | $HCl \cdot 2H_2O$ | 156-158 |
| OAc | 1 | $N(C_2H_5)_2$ | $HCl \cdot 1,5H_2O$ | 175-177 |
| OAc | 1 | $N-[CH(CH_3)_2]_2$ | $HCl \cdot H_2O$ | 152-154 |
| OAc | 1 | 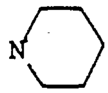 | HCl | 233-235 |
| OAc | 1 | 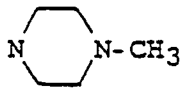 | 2HCl | 176-178 |
| OAc | 1 | 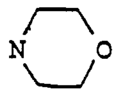 | HCl | 173-175 |
| OAc | 2 | $N(CH_3)_2$ | $HCl \cdot 1,5H_2O$ | 173-175 |
| OAC | 3 | $N(C_2H_5)_2$ | HCl | 233-235 |
| OH | 1 | 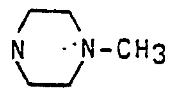 | $HCl \cdot 1,5H_2O$ | 161-162 |
| OH | 1 | 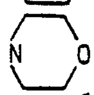 | $HCl \cdot 2,5H_2O$ | 168-170 |
| OH | 1 | 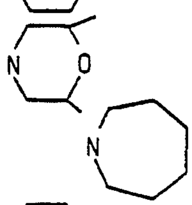 | HCl | 142-143 |
| OH | 1 |  | $HCl \cdot H_2O$ | 134-136 |
| OH | 1 | 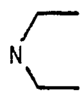 | $HCl \cdot H_2O$ | 145-146 |
| OH | 1 | 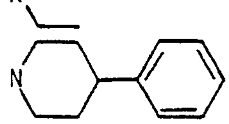 | $HCl \cdot 0,5H_2O$ | 155-156 |
| OH | 1 | 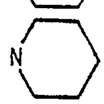 | HCl | 149-150 |
| OH | 2 | $N(CH_3)_2$ | HCl | 132-133 |
| OAc | 1 | 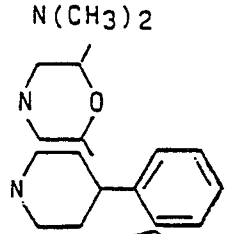 | HCl | 146-148 |
| OAc | 1 |  | HCl | 140-141 |
| OAc | 1 | 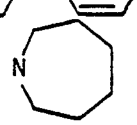 | $HCl \cdot 0,5H_2O$ | 221 |

| $R_7$ | m' | $R_{23}$ | X | Schmp. (°C) |
|---|---|---|---|---|
| OAc | 2 | $NH_2$ | $HCl \cdot 2H_2O$ | 157 |
| OAc | 2 | $N[(CH_2)_4CH_3]_2$ | HCl | 105-107 |
| OAc | 2 | 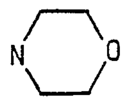 | $HCl \cdot 2,5H_2O$ | 228-230 |
| OAc | 2 | 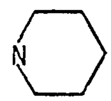 | $HCl \cdot 0,5H_2O$ | 231-233 |
| OAc | 2 | 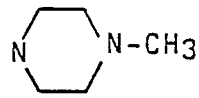 | $2HCl \cdot 3H_2O$ | 175-177 |
| OAc | 2 | 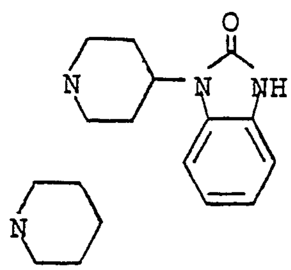 | $HCl \cdot 1,5H_2O$ | 195-197 |
| OAc | 3 | | $HCl \cdot H_2O$ | 134-135 |
| OAc | 3 | 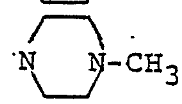 | $2HCl \cdot H_2O$ | 168-169 |

Gegenstand der Erfindung ist auch ein Verfahren zur Herstellung der Verbindungen der allgemeinen Formel I.

Das Verfahren ist dadurch gekennzeichnet, daß man eine Verbindung der Formel IV

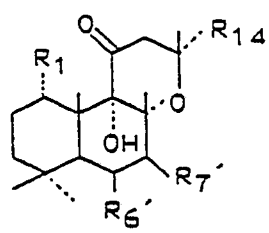

IV

worin $R_1$ die zur Formel I angegebenen Bedeutungen hat, $R_{14}$ eine Vinyl-, Ethyl-, Cyclopropyl-Gruppe oder die Gruppe $-C\overset{Z}{C}=CH_2$ darstellt, $R_6'$ die OH-Gruppe und $R_7'$ eine Rest der Formel V

$$-O-\overset{A}{\overset{\|}{C}}-(\overset{R_{15}}{\underset{R_{16}}{C}})_l-(CH_2)_m-(\overset{R_{17}}{\underset{R_{18}}{C}})_n-OH \qquad (V)$$

oder die Acetylgruppe oder $R_7'$ die OH-Gruppe und $R_6'$ einen Rest der Formel V darstellt,

a) mit einer Verbindung der Formel VI

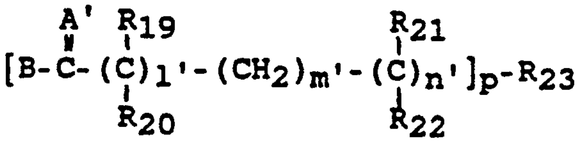

(VI)

worin B, A′, $R_{19}$-$R_{23}$, l′, m′, n′ und p die genannten Bedeutungen haben,

b) falls $R_{23}$ Halogen bedeutet, das erhaltene Kondensationsprodukt mit einer Verbindung der Formel $HNR_{24}R_{25}$, worin $R_{24}$ und $R_{25}$ die genannten Bedeutungen haben, umsetzt, und

c) falls $R_{23}$ eine geschützte Aminogruppe darstellt, die Schutzgruppe nach üblichen Methoden abspaltet.

Eine bevorzugte Ausführungsform des genannten Verfahrens ist die, worin Verbindungen der Formel IV, worin $R_1$ die OH-Gruppe, $R_{14}$ die Vinylgruppe, entweder $R_6'$ die OH-Gruppe und $R_7'$ einen Rest der Formel

$-O-\overset{O}{\overset{\|}{C}}-CH_2OH$, oder $R_7'$ die OH-Gruppe und $R_6'$ einen Rest der Formel

$-O-\overset{O}{\overset{\|}{C}}-CH_2-OH$ darstellt, mit einer Verbindung der Formel VI

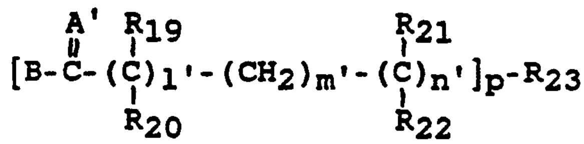

(VI)

worin B die OH-Gruppe, A′ Sauerstoff, l′ und n′ jeweils Null, m′ eine ganze Zahl von 1 bis 10 bedeuten und $R_{23}$ die Gruppe $NR_{24}R_{25}$ darstellt, worin $R_{24}$ und $R_{25}$ die obengenannten Bedeutungen haben, umgesetzt werden.

Falls R eine geschützte Aminogruppe darstellt, wird das Kondensat einer Reaktion unterworfen, die die Schutzgruppe von der Aminogruppe entfernt, um zu einer erfindungsgemäßen Verbindung zu gelangen. Im Falle von Verbindungen, worin $R_{23}$ für Halogen steht, wird das Kondensat mit Aminen der Formel $HNR_{24}R_{25}$, worin $R_{24}$ und $R_{25}$ die gleiche Bedeutung wie oben beschrieben haben, weiter behandelt.

Falls die Carbonsäuren der allgemeinen Formel VI als solche in den obigen Kondensationen verwendet werden, werden die gewünschten Ergebnisse erhalten, wenn die Kondensation in einem Lösungsmittel, wie Ethylacetat, Ether, Chloroform, in Gegenwart von Dicyclohexylcarbodiimid, oder Dicyclohexylcarbodiimid und 4-Dimethylaminopyridin, oder Carbonyldiimidazol, während einer Periode von 0,5 bis 48 Stunden und bei Temperaturen üblicherweise zwischen 0° C und Siedepunkt des verwendeten Lösungsmittels durchgeführt wird.

Im Falle der obigen Kondensationsprodukte, die unter Verwendung von Verbindungen der Formel VI, worin $R_{23}$ eine geschützte Aminogruppe, z.B. Tritylamino, ist, erhalten werden, wird die Entfernung der Schutzgruppe z.B. durch Verwendung von Trifluoressigsäure, durchgeführt.

Falls Kondensationsprodukte durch Umsetzen mit einer Verbindung der Formel VI, worin $R_{23}$ Halogen ist, erhalten werden, wird weiter mit einem Amin der allgemeinen Formel $HNR_{24}R_{25}$ umgesetzt. Die Umsetzung wird durchgeführt in einem Lösungsmittel, wie Toluol, Chloroform, Dichlormethan oder dem benützten Amin unter Rühren während einer Periode von 0,5 bis 24 Stunden und bei Temperaturen im Bereich von 0° C bis zum Siedepunkt des Lösungsmittels.

Eine Verbindung der allgemeinen Formel VI, worin $R_{23}$ für eine geschützte Aminogruppe steht, ist z.B. Tritylglycin. Ein Beispiel einer Verbindung der Formel VI, worin $R_{23}$ für $NHR_{24}R_{25}$ steht, ist Dimethylaminopropionsäure-hydrochlorid. Beispiele für Amine der allgemeine Formel $HNR_{24}R_{25}$ sind Pyrrolidin, N-Methylpiperazin, Piperidin, Morpholin.

Gewünschtenfalls werden die Säureadditionssalze von Verbindungen der Erfindung hergestellt, indem die Lösung von Verbindungen in organischen Lösungsmitteln, wie Ether, mit der etherischen Lösung einer Säure, z.B. Chlorwasserstoffsäure, behandelt werden.

Die als Ausgangsverbindungen in den hier beschriebenen Verfahren benützten Verbindungen der Formel IV werden durch Methoden erhalten, wie sie in der deutschen Patentanmeldung P 37 18 589.6 beschrieben sind, wobei diese Methoden gegebenenfalls kleinen Modifikationen unterliegen, wie sie für den

Fachmann bekannt sind.

Die Verbindungen der Vorliegenden Erfindung und ihre Salze weisen pharmakologische Eigenschaften auf, die der Klasse von polyoxylierten Labdanen und ihren Derivaten zukommen. Sie zeigen aber in spezifischerer Weise eine selektive Wirkung hinsichtlich der positiv inotropen Aktivität, antihypertensiven Aktivität und Verminderung des intraokularen Drucks.

Dies wird durch die folgenden pharmakologischen Versuchsergebnisse gezeigt.

**Positive inotrope Wirkung:**

Die folgende Methode wurden angewendet.

400 g schwere Meerschweinchen beiderlei Geschlechts werden getötet, das Herz entnommen und bei Raumtemperatur in eine Ringerlösung gebracht. Sowohl der linke als auch der rechte Vorhof werden dann isoliert, an einem Organhalter befestigt und das Präparat in eine Ringer-Lösung enthaltendes, bei einer Temperatur von 32 °C gehaltenes Organbad gebracht. Man läßt dann ein Gemisch von 95 % $O_2$ und 5% $CO_2$ durch das Organbad perlen und stimuliert den Vorhof elektrisch. Die zu prüfende Verbindung wird in Wasser zu einer Lösung bekannter Konzentration gelöst, und dem Bad zugesetzt. Die Kontraktionsstärke des Vorhofs wird während 7 bis 10 Minuten auf einem 4-Kanal Nihon Kohden Schreiber mit isometrischem Dehnungsmeßstreifen registriert. Die Aktivität wird aus den erhaltenen Daten als $EC_{50}$ ausgedrückt.

Die erhaltenen Resultate in diesem Modell werden in den folgenden Tabellen III und IV für repräsentative Verbindungen der Erfindung angegeben:

**Tabelle III**

| Verbindung | | Meerschweinchen-Vorhof $EC_{50}$ μg/ml |
|---|---|---|
| 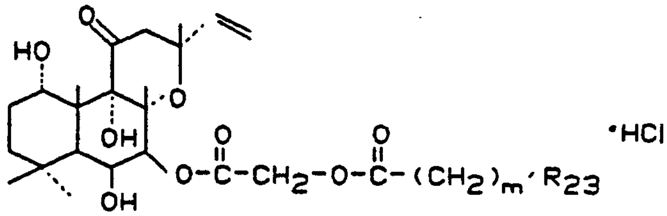 Ia | | |
| m' | $R_{23}$ | |
| 1 | $NH_2$ | 0,1 |
| 1 | 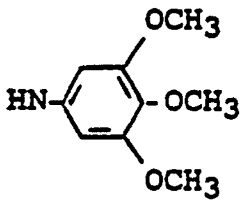 | 0,545 |
| 1 | $N[CH(CH_3)_2]_2$ | 1,0 |
| 1 | $N(C_2H_5)_2$ | 0,03 |
| 1 | 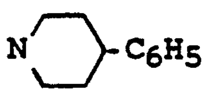 | 0,18 |
| 1 | 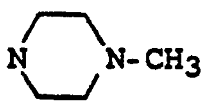 | 0,1 |
| 1 | 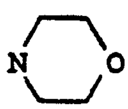 | 1,42 |
| 1 | 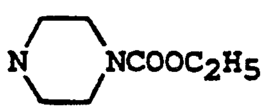 | >1,0 |
| 1 | $N(CH_3)_2$ | 0,3 |

Tabelle IV

Verbindung

Meerschweinchen Vorhof
$EC_{50}$ µg/ml

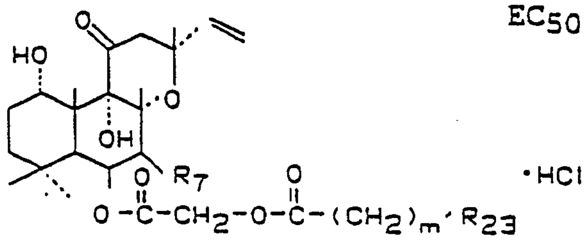

Ib

| m' | $R_7$ | $R_{23}$ | |
|---|---|---|---|
| 1 | OH | $NH_2$ | >10 |
| 1 | OAc | $NH_2$ | 0,008 |
| 1 | OAc | $N(C_2H_5)_2$ | 0,03 |
| 1 | OAc | $N[CH(CH_3)_2]_2$ | 0,14 |
| 1 | OAc | 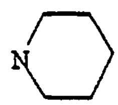 | 0,04 |
| 1 | OAc | 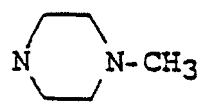 | 0,006 |
| 1 | OAc | 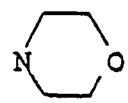 | 0,072 |
| 2 | OAc | $N(CH_3)_2$ | 0,058 |
| 2 | OAc | $NH_2$ | 0,02 |
| 2 | OAc | 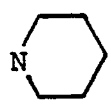 | 0,086 |

Prüfung auf antihypertensive Wirkung

Blutdruck bei Katzen

3 bis 4 kg schwere Katzen beiderlei Geschlechts werden mit Ether anästhesiert und unter Chloralose-Anästhesie (70 mg/kg, i.v.) gehalten. Die Femoral-Arterie und -Vene werden zur Aufzeichnung des Blutdrucks bzw. zur Verabreichung der Arzneimittel mit Kanülen versehen. Der Blutdruck der Femoralarterie wird mittels eines Statham P 23 Db Druckaufnehmers auf einem Nihon-Kohden Schreiber für physiologische Zwecke ausgezeichnet. Die zu prüfende Verbindung wird in destilliertem Wasser aufgelöst und intravenös verabreicht. Es wurden der Blutdruckabfall und die Dauer der blutdrucksenkenden Wirkung bestimmt.

Die in diesem Modell erhaltenen Ergebnisse werden für repräsentative Verbindungen der Erfindung in der folgenden Tabelle V angegeben:

**Tabelle V**

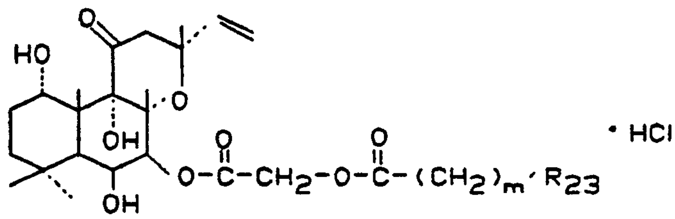

Ia

| m' | $R_{23}$ | Dosis (mg/kg) | Abfall des BD (mm Hg) | Dauer (min) |
|---|---|---|---|---|
| 1 | $NH_2$ | 0,1 | 20 | 120 |
| 1 | NHBoC | 0,3 | 30 | 35 |
| 1 | $NH[CH(CH_3)_2]_2$ | 0,1 | 25 | 125 |
| 1 | 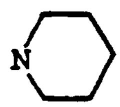 | 0,1 | 25 | 40 |
| 1 | 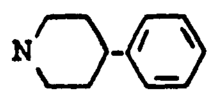 | 1 | 60 | 45 |
| 1 | 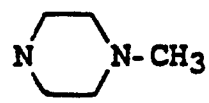 | 0,1 | 20 | 40 |
| 1 | 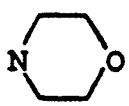 | 0,1 | 30 | 60 |
| 1 | 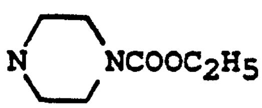 | 0,3 | 35 | 30 |
| 1 | 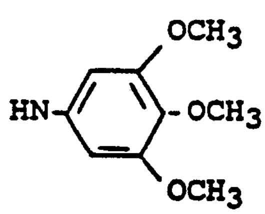 | 1 | 80 | 60 |

Die folgenden Beispiele veranschaulichen die Erfindung, schränken aber den Umfang der Erfindung nicht ein.

**BEISPIEL 1**

**8,13-Epoxy-1α,6β,9α-trihydroxy-7β-(2-N-tritylglycinyloxy)-acetoxy-labd-14-en-11-on**

Tritylglycin (0,087 g; 0,275 mmol) in Dimethylformamid (0,5 ml) wurden zu einer Lösung von 8,13-Epoxy-7β-(2-hydroxyacetoxy)-1α,6β,9α-trihydroxy-labd-14-en-11-on (0,107 g; 0,25 mmol) und Dicyclohexylcarbodiimid (0,062 g; 0,3 mmol) in Ethylacetat (5 ml) unter starken Rührbedingungen bei Raumtemperatur zugesetzt. Nach 15 Minuten wurde 4-Dimethylaminopyridin (0,031 g; 0,25 mmol) zugesetzt und die Mischung wurde 16 Stunden gerührt. Die Reaktionsmischung wurde filtriert und das Filtrat wurde mit Kochsalzlösung gewaschen und über $Na_2SO_4$ getrocknet. Das Lösungsmittel wurde entfernt und das zurückbleibende Öl wurde durch "Flash"-Chromatographie unter Verwendung von 20 % Ethylacetat-Petrolether gereinigt. Ausbeute 0,135 g (74,5 %).

Entsprechend dem oben beschriebenen Verfahren wurde aus 8,13-Epoxy-6β-(2-hydroxyacetoxy)-1α,7β-9α-trihydroxy-labd-14-en-11-on die Verbindung 8,13-Epoxy-6β-(2-N- tritylglycinyloxy)acetoxy-1α,7β,9α-trihydroxy-labd-14-en-11-on in 75 %iger Ausbeute hergestellt.

Die Verbindung 7β-Acetoxy-1α,9α-dihydroxy-8,13-epoxy-6β-(2-N-tritylglycinyloxy)acetoxy-labd-14-en-11-on wurde aus 7β-Acetoxy-1α,9α-dihydroxy-8,13-epoxy-6β-(2-hydroxy-acetoxy)-labd-14-en-11-on in 61 %iger Ausbeute hergestellt.

**BEISPIEL 2**

**8,13-Epoxy-7β-(2-glycinyloxy)acetoxy-1α,6β,9α-trihydroxy-labd-14-en-11-on-monohydrochlorid**

8,13-Epoxy-1α,6β,9α-trihydroxy-7β-(2-N-tritylglycinyloxy)-acetoxy-labd-14-en-11-on (0,1 g; 0,138 mmol) wurde in Ether (10 ml) gelöst und dazu wurde Trifluoressigsäure (0,2 ml) im Überschuß zugesetzt. Die klare Lösung wurde während 1 Stunde bei Raumtemperatur gehalten. Dazu wurde HCl/Ether zugesetzt. Der entstehende weiße Niederschlag wurde filtriert, mit trockenem Ether gewaschen und schließlich im Hochvakuum getrocknet. Ausbeute 0,052 g (72,57 %), Schmp. 167 bis 72°.

Gemäß dem oben beschriebenen Verfahren wurde die Verbindung 8,13-Epoxy-6β-(2-glycinyloxy)-acetoxy-1α,7β,9α-trihydroxy-labd-14-en-11-on-hydrochlorid, Schmp. 165-167° C, aus 8,13-Epoxy-6β-(2-N-tritylglycinyloxy)acetoxy-1α,7β,9α-trihydroxy-labd-14-en-11-on erhalten.

Die Verbindung 7β-Acetoxy-8,13-epoxy-6β-(2-glycinyloxy)-acetoxy-1α,9α-dihydroxy-labd-14-en-11-on-hydrochlorid, Schmp. 181-183° C, wurde aus 7β-Acetoxy-8,13-epoxy-6β-(2-N-tritylglycinyloxy)acetoxy-1 α,9α-dihydroxy-labd-14-en-11-on erhalten.

**BEISPIEL 3**

**7β-(2-Dimethylaminopropionyloxy)acetoxy-8,13-epoxy-1α,6β,9α-trihydroxy-labd-14-en-11-on-monohydrochlorid-dihydrat**

8,13-Epoxy-7β-(2-hydroxyacetoxy)-1α,6β,9α-trihydroxy-labd-14-en-11-on (0,4264 g; 1 mmol) und Dicyclohexylcarbodiimid (0,21 g; 1,05 mmol) wurden in Ethylacetat (5 ml) gelöst und dazu wurde unter starkem Rühren bei Raumtemperatur β-Dimethylaminopropionsäurehydrochlorid (0,153 g; 1 mmol) in Dimethylformamid (1,4 ml) zugesetzt. Nach 10 Minuten wurde 4-Dimethylaminopyridin (0,185 g; 1,5 mmol) zugesetzt, und die Reaktionsmischung wurde unter Rühren während 21 Stunden bei Raumtemperatur gehalten. Die Reaktionsmischung wurde mit Ether verdünnt und filtriert. Das Filtrat wurde mit Kochsalzlösung gewaschen und über wasserfreiem $Na_2SO_4$ getrocknet. Das Lösungsmittel wurde unter vermindertem Druck entfernt. Die Mischung wurde dann durch "Flash"-Chromatographie unter Verwendung von 20 % Acetonitril in Chloroform, gefolgt von 10 % Methanol in Chloroform gereinigt. Das gereinigte Material wurde in Ethylacetat gelöst und durch Zusatz von HCl/Ether in das Hydrochlorid übergeführt, filtriert und mit trockenem Ether und schließlich mit trockenem Ethylacetat gewaschen. Es wurde dann im Hochvakuum getrocknet. Ausbeute 0,12 g (21 %); Schmp. 214-15° C.

**BEISPIEL 4**

**7β-(2-Chloracetyloxy)acetoxy-8,13-epoxy-1α,6β,9α-trihydroxy-labd-14-en-11-on**

Zu einer Lösung von 8,13-Epoxy-7β-(2-hydroxyacetoxy)-1α,6β,9α-trihydroxy-labd-14-en-11-on (10,08 g; 24 mmol) und Dicyclohexylcarbodiimid (6,53 g; 31,68 mmol) in trockenem Ethylacetat (125 ml) wurde unter Rühren bei Raumtemperatur Chloressigsäure (2,72 g; 28,8 mmol) in Ethylacetat (25 ml) zugesetzt. Nach 15 Minuten wurde 4-Dimethylaminopyridin (2,93 g; 24 mmol) zugesetzt und das Rühren während 3 Stunden fortgesetzt. Die Reaktionsmischung wurde filtriert und das Filtrat mit Kochsalzlösung gewaschen und über wasserfreiem $Na_2SO_4$ getrocknet. Nach Entfernung des Lösungsmittels wurd der Rückstand durch "Flash"-Chromatographie gereinigt. Ausbeute 5 g (49,75 %), Schmp. 168-70° C.

**BEISPIEL 5**

**8,13-Epoxy-7β-(2-diisopropylaminoacetyloxy)acetoxy-1α,6β,9α-trihydroxy-labd-14-en-11-on-hyrochlorid-sesquihydrat**

7β-(2-Chloracetyloxy)acetoxy-8,13-epoxy-1α,6β-9α-trihydroxy-labd-14-en-11-on (0,25 g; 0,5 mmol) und Diisopropylamin (2 ml) wurden 3 Stunden zum Rückfluß erhitzt. Überschüssiges Amin wurde dann unter vermindertem Druck entfernt und die zurückbleibende Mischung wurde durch "Flash"-Chromatographie unter Verwneudng von Acetonitril in Chloroform gereinigt. Das gereinigte Material wurde in Ether gelöst und HCl/Ether wurde zugesetzt. Der erhaltene Feststoff wurde abfiltriert und mit trockenem Ether gewaschen und getrocknet. Ausbeute 0,12 g (39,8 %), Schmp. 148-50° C.

**BEISPIEL 6**

**8,13-Epoxy-1α,6β,9α-trihydroxy-7β-[2-(3,4,5-trimethoxyanilino)acetyloxy]-acetoxy-labd-14-en-11-on**

7β-(2-Chloracetyloxy)acetoxy-8,13-epoxy-1α,6β,9α-trihydroxy-labd-14-en-11-on (0,25 g; 0,5 mmol) wurde in Toluol (3 ml) gelöst und dazu wurde 3,4,5-Trimethoxyanilin zugesetzt. Die Mischung wurde 16 Stunden auf 70 bis 80° C erhitzt. Das Lösungsmittel wurde unter vermindertem Druck entfernt und der Rückstand wurde durch "Flash"-Chromatographie unter Verwendung von Acetonitril in Chloroform gereinigt. Ausbeute 0,06 g (17,5 %), Schmp. 108-109° C.

Gemäß dem oben beschriebenen Verfahren wurden unter Verwendung des geeigneten Amins anstelle von Diisopropylamin die folgenden Verbindungen hergestellt:
8,13-Epoxy-7β-(2-diallylaminoacetoxy)acetoxy-1α,6β,9α-trihydroxy-labd-14-en-11-on-hydrochlorid, Schmp. 135-36° C.
8,13-Epoxy-7β-(2-N,N-diethylglycinyloxy)acetoxy-1α,6β,9α-trihydroxy-labd-14-en-11-on-hydrochlorid, Schmp. 140-42° C.
8,13-Epoxy-7β-(2-piperidinoacetoxy)acetoxy-1α,6β,9α-trihydroxy-labd-14-en-11-on-hydrochlorid, Schmp. 103-04° C.
8,13-Epoxy-7β-[2-(4-phenylpiperidinoacetoxy)]acetoxy-1α,6β,9α-trihydroxy-labd-14-en-11-on-hydrochlorid, Schmp. 99-101° C.
8,13-Epoxy-7β-(2-homopiperidinoacetoxy)acetoxy-1α,6β,9α-trihydroxy-labd-14-en-11-on-hydrochloridsesquihydrat, Schmp. 146-48° C.
8,13-Epoxy-7β-(2-N-methylpiperazinoacetoxy)acetoxy-1α,6β,9α-trihydroxy-labd-14-en-11-on, Schmp. 95-97° C.
8,13-Epoxy-7β-(2-morpholinoacetoxy)acetoxy-1α,6β,9α-trihydroxy-labd-14-en-11-on, Schmp. 82° C.
8,13-Epoxy-7β-[2-(2,6-dimethylmorpholinoacetoxy)acetoxy]-1α,6β,9α-trihydroxy-labd-14-en-11-on-hydrochlorid, Schmp. 158-60° C.
8,13-Epoxy-7β-[2-(N-carbethoxypiperazinoacetoxy)acetoxy]-1α,6β,9αtrihydroxy-labd-14-en-11-on-hydrochlorid, Schmp. 157-58° C.

In ähnlicher Weise wurden gemäß dem oben beschriebenen Verfahren unter Verwendung von 7β-Acetoxy-6β-(2-chloracetyloxy)acetoxy-8,13-epoxy-1α,9α-dihydroxy-labd-14-en-11-on anstelle von 7β-(2-Chloracetyloxy)acetoxy-8,13-epoxy-1α,6β,9α-trihydroxy-labd-14-en-11-on und dem geeigneten Amin anstelle von Diisopropylamin die folgenden Verbindungen hergestellt:
7β-Acetoxy-6β-[2-(N,N-dimethylaminoacetoxy)acetoxy]-8,13-epoxy-1α,9α-dihydroxy-labd-14-en-11-on-hydrochlorid-dihydrat, Schmp. 156-58° C.

7β-Acetoxy-6β-(2-N,N-diethylaminoacetoxy)acetoxy-8,13-epoxy-1α,9α-dihydroxy-labd-14-en-11-on-hydrochloridsesquihydrat, Schmp. 175-77° C.
7β-Acetoxy-6β-(2-N,N-diisopropylaminoacetoxy)acetoxy-8,13-epoxy-1α,9α-dihydroxy-labd-14-en-11-on-hydrochloridmonohydrat, Schmp. 152-54° C.
7β-Acetoxy-6β-(2-piperidinoacetoxy)acetoxy-1α,9α-dihydroxy-8,13-epoxy-labd-14-en-11-on-hydrochlorid, Schmp. 233-35° C.
7β-Acetoxy-6β-(2-N-methylpiperazinoacetoxy)acetoxy-8,13-epoxy-1α,9α-dihydroxy-labd-14-en-11-on-dihydrochlorid, Schmp. 176-78° C.
7β-Acetoxy-6β-(2-morpholinoacetoxy)acetoxy-1α,9α-dihydroxy-8,13-epoxy-labd-14-en-11-on-hydrochlorid.

## Ansprüche

1. Verbindungen der Formel I

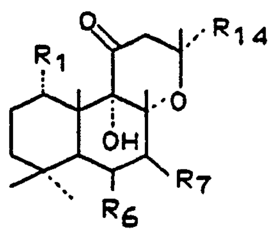

I

worin bedeuten
$R_1$ OH, O-Alkyl oder einen Rest der Formel II

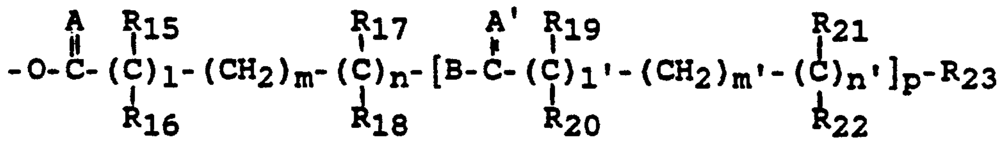

II

worin A und A′ für Sauerstoff oder Schwefel, B für $-CH_2-$, Sauerstoff, Schwefel oder -NH-, $R_{15}$-$R_{23}$ für Wasserstoff, Alkyl, Aryl, Aralkyl, Hydroxy, Alkoxy, Thiol, Halogen oder eine Gruppe der Formel $NR_{24}R_{25}$ stehen, worin $R_{24}$ und $R_{25}$, falls sie gleich sind, Wasserstoff, Alkyl, substituiertes Alkyl, Aryl oder Aralkyl bedeuten, oder falls $R_{24}$ für Wasserstoff steht, $R_{25}$ Alkyl, substituiertes Alkyl, Cycloalkyl, Aralkyl, Aryl, einen Heterocyclus, Amino, Dialkylamino, Alkylamino, Arylamino, Aralkylamino, Hydroxy, Thiol, Acyloxy, Acyl, Carbamoyl, Carboxyalkyl, Carbalkoxyalkyl, Dialkylaminoalkyl bedeutet, oder falls $R_{24}$ für Alkyl steht, $R_{25}$ substituiertes Alkyl, Cycloalkyl, Aryl, Aralkyl oder Dialkylaminoalkyl bedeutet, oder $R_{24}$ und $R_{25}$ zusammen mit dem Stickstoffatom, an das sie gebunden sind, für einen Heterocyclus stehen, der ein oder mehrere Heteroatome aufweisen und gegebenenfalls ein- oder mehrfach mit Alkyl, Aryl, Hydroxyalkyl, Halogen, Hydroxy, Alkoxy oder anderen heterocyclischen Gruppen substituiert sein kann, mit der Maßgabe, daß der Rest mindestens drei der Substituenten $R_{15}$-$R_{23}$ enthält, wobei mindestens einer der drei Substituenten ein Heteroatom der Gruppe N, O oder S aufweist,
und
l, m, n, l′, m′, n′ jeweils 0 oder eine ganze Zahl von 1 bis 10 und p eine ganze Zahl von 1 bis 10 bedeuten,
$R_6$ OH, O-Alkyl oder eine Gruppe der Formel II

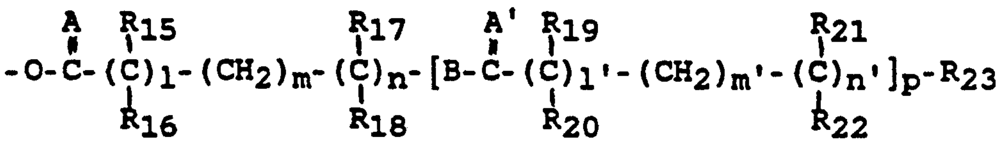

II

worin A, A′, l, m, n, l′, m′, n′, p und $R_{15}$-$R_{23}$ die gleiche Bedeutung wie oben angegeben haben,

$R_7$ OH, O-Alkyl oder eine Gruppe der Formel II

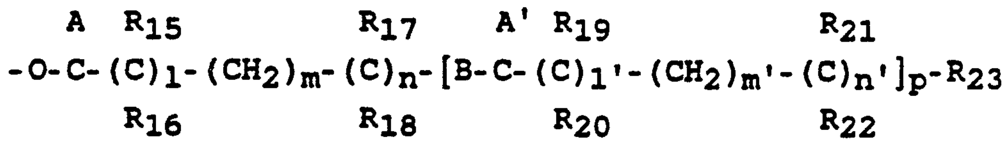

II

worin A, A', l, m, n, l', m', n', p und $R_{15}$-$R_{23}$ die gleiche Bedeutung wie oben angegeben haben, $R_{14}$ Vinyl, Ethyl, Cyclopropyl, $CHOHCH_2OH$, $CH_2OH$ oder $-\overset{Z}{\overset{|}{C}}=CH_2$, worin Z für Halogen, wie Chlor, Brom oder Fluor steht, mit der Maßgabe, daß

a) $R_1$, $R_6$ und $R_7$ nicht gleichzeitig OH-Gruppen sind,

b) falls $R_7$ $O-\overset{O}{\overset{\|}{C}}-CH_3$ ist, $R_1$ und $R_6$ nicht OH sind,

sowie deren pharmakologisch unbedenklichen Säureadditionssalze.

2. Verbindungen der Formel I gemäß Anspruch 1, dadurch gekennzeichnet, daß $R_1$ die OH-Gruppe und $R_{14}$ die Vinylgruppe bedeutet, einer der beiden Substituenten $R_6$ und $R_7$ OH, O-Alkyl oder O-Acyl bedeutet und der andere einen Rest der Formel

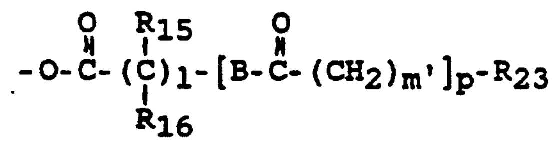

darstellt, worin $R_{15}$, $R_{16}$, $R_{23}$, B, l und m' die angegebene Bedeutung haben und p= 1 ist, sowie deren pharmakologisch unbedenklichen Säureadditionssalze.

3. Verbindungen gemäß Anspruch 2, dadurch gekennzeichnet, daß $R_{15}$ und $R_{16}$ jeweils Wasserstoff, B Sauerstoff, l die Zahl 1, m' eine ganze Zahl von 1 bis 4 bedeuten und $R_{23}$ einen Rest der Formel $NR_{24}R_{25}$ darstellt, wobei $R_{24}$ und $R_{25}$ die genannten Bedeutungen haben, sowie deren pharmakologisch unbedenklichen Säureadditionssalze.

4. Verbindungen gemäß Anspruch 3, dadurch gekennzeichnet, daß einer der beiden Substituenten $R_6$ und $R_7$ die OH- oder Acetylgruppe bedeutet, sowie deren pharmakologisch unbedenklichen Säureadditionssalze.

5. Arzneimittel, gekennzeichnet durch einen Gehalt an einer Verbindung gemäß Anspruch 1 und pharmazeutisch unbedenklichen Hilfs- und/oder Trägerstoffen.

6. Verwendung einer Verbindung gemäß Anspruch 1 zur Herstellung eines Arzneimittels mit positiv inotropen und/oder blutdrucksenkender Wirkung.

7. Verwendung einer Verbindung gemäß Anspruch 1 zur Herstellung eines Arzneimittels mit Augeninnendruck-senkender Wirkung.

8. Verfahren zur Herstellung einer Verbindung der Formel I gemäß Anspruch 1, dadurch gekennzeichnet, daß man eine Verbindung der Formel IV

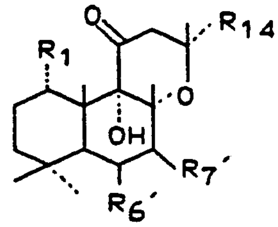

IV

worin $R_1$ die zur Formel I angegebenen Bedeutungen hat, $R_{14}$ eine Vinyl-, Ethyl-, Cyclopropyl-Gruppe oder die Gruppe

$-\overset{Z}{\underset{|}{C}}=CH_2$ darstellt, $R_6'$ die OH-Gruppe und $R_7'$ eine Rest der Formel V

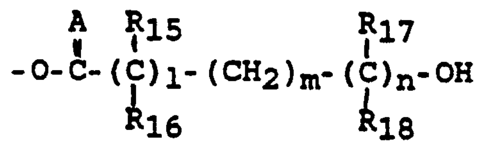

(V)

oder die Acetylgruppe oder $R_7'$ die OH-Gruppe und $R_6'$ einen Rest der Formel V darstellt,

a) mit einer Verbindung der Formel VI

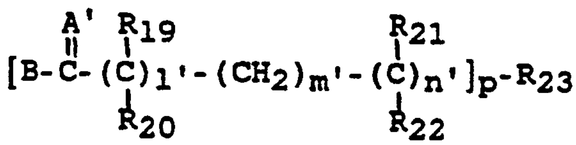

(VI)

worin B, A′, $R_{19}$-$R_{23}$, l′, m′, n′ und p die genannten Bedeutungen haben, umsetzt

b) falls $R_{23}$ Halogen bedeutet, das erhaltene Kondensationsprodukt mit einer Verbindung der Formel $HNR_{24}R_{25}$, worin $R_{24}$ und $R_{25}$ die genannten Bedeutungen haben, umsetzt, und

c) falls $R_{23}$ eine geschützte Aminogruppe darstellt, die Schutzgruppe nach üblichen Methoden abspaltet.

Patentansprüche für folgende Vertragsstaaten: ES, GR

1. Verfahren zur Herstellung von Verbindungen der Formel I

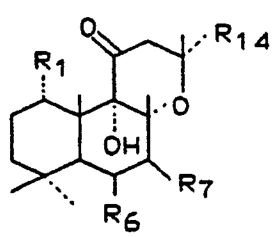

I

worin bedeuten

$R_1$ OH, O-Alkyl oder einen Rest der Formel II

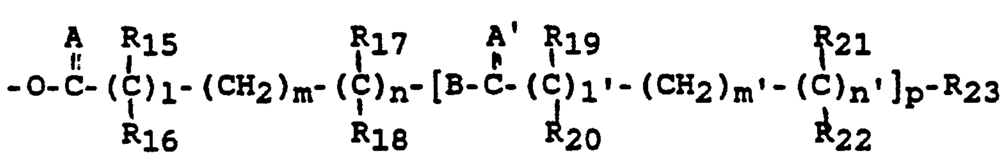

II

worin A und A′ für Sauerstoff oder Schwefel, B für $-CH_2-$, Sauerstoff, Schwefel oder -NH-, $R_{15}$-$R_{23}$ für Wasserstoff, Alkyl, Aryl, Aralkyl, Hydroxy, Alkoxy, Thiol, Halogen oder eine Gruppe der Formel $NR_{24}R_{25}$ stehen, worin $R_{24}$ und $R_{25}$, falls sie gleich sind, Wasserstoff, Alkyl, substituiertes Alkyl, Aryl oder Aralkyl bedeuten, oder falls $R_{24}$ für Wasserstoff steht, $R_{25}$ Alkyl, substituiertes Alkyl, Cycloalkyl, Aralkyl, Aryl, einen Heterocyclus, Amino, Dialkylamino, Alkylamino, Arylamino, Aralkylamino, Hydroxy, Thiol, Acyloxy, Acyl,

Carbamoyl, Carboxyalkyl, Carbalkoxyalkyl, Dialkylaminoalkyl bedeutet, oder falls $R_{24}$ für Alkyl steht, $R_{25}$ substituiertes Alkyl, Cycloalkyl, Aryl, Aralkyl oder Dialkylaminoalkyl bedeutet, oder $R_{24}$ und $R_{25}$ zusammen mit dem Stickstoffatom, an das sie gebunden sind, für einen Heterocyclus stehen, der ein oder mehrere Heteroatome aufweisen und gegebenenfalls ein- oder mehrfach mit Alkyl, Aryl, Hydroxyalkyl, Halogen, Hydroxy, Alkoxy oder anderen heterocyclischen Gruppen substituiert sein kann, mit der Maßgabe, daß der Rest mindestens drei der Substituenten $R_{15}$-$R_{23}$ enthält, wobei mindestens einer der drei Substituenten ein Heteroatom der Gruppe N, O oder S aufweist,

und

l, m, n, l′, m′, n′ jeweils 0 oder eine ganze Zahl von 1 bis 10 und p eine ganze Zahl von 1 bis 10 bedeuten,

$R_6$ OH, O-Alkyl oder eine Gruppe der Formel II

$$-O-\overset{\overset{A}{\|}}{C}-(\underset{R_{16}}{\overset{R_{15}}{C}})_l-(CH_2)_m-(\underset{R_{18}}{\overset{R_{17}}{C}})_n-[B-\overset{\overset{A'}{\|}}{C}-(\underset{R_{20}}{\overset{R_{19}}{C}})_{l'}-(CH_2)_{m'}-(\underset{R_{22}}{\overset{R_{21}}{C}})_{n'}]_p-R_{23} \qquad II$$

worin A, A′, l, m, n, l′, m′, n′, p und $R_{15}$-$R_{23}$ die gleiche Bedeutung wie oben angegeben haben,

$R_7$ OH, O-Alkyl oder eine Gruppe der Formel II

$$-O-\overset{\overset{A}{\|}}{C}-(\underset{R_{16}}{\overset{R_{15}}{C}})_l-(CH_2)_m-(\underset{R_{18}}{\overset{R_{17}}{C}})_n-[B-\overset{\overset{A'}{\|}}{C}-(\underset{R_{20}}{\overset{R_{19}}{C}})_{l'}-(CH_2)_{m'}-(\underset{R_{22}}{\overset{R_{21}}{C}})_{n'}]_p-R_{23} \qquad II$$

worin A, A′, l, m, n, l′, m′, n′, p und $R_{15}$-$R_{23}$ die gleiche Bedeutung wie oben angegeben haben,

$R_{14}$ Vinyl, Ethyl, Cyclopropyl, $CHOHCH_2OH$, $CH_2OH$ oder

$-\overset{\overset{Z}{|}}{C}=CH_2$, worin Z für Halogen, wie Chlor, Brom oder Fluor steht,

mit der Maßgabe, daß

a) $R_1$, $R_6$ und $R_7$ nicht gleichzeitig OH-Gruppen sind,

b) falls $R_7$

$O-\overset{\overset{O}{\|}}{C}-CH_3$ ist, $R_1$ und $R_6$ nicht OH sind,

sowie deren pharmakologisch unbedenklichen Säureadditionssalzen, dadurch gekennzeichnet, daß man eine Verbindung der Formel IV

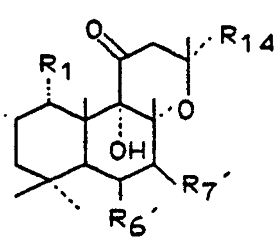

IV

worin $R_1$ die zur Formel I angegebenen Bedeutungen hat, $R_{14}$ eine Vinyl-, Ethyl-, Cyclopropyl-Gruppe oder die Gruppe

$-\overset{\overset{Z}{|}}{C}=CH_2$ darstellt, $R_6'$ die OH-Gruppe und $R_7'$ eine Rest der Formel V

$$-O-\overset{A}{\overset{\|}{C}}-(\overset{R_{15}}{\underset{R_{16}}{C}})_{l}-(CH_2)_m-(\overset{R_{17}}{\underset{R_{18}}{C}})_n-OH \qquad (V)$$

oder die Acetylgruppe oder $R_7'$ die OH-Gruppe und $R_6'$ einen Rest der Formel V darstellt,

a) mit einer Verbindung der Formel VI

$$[B-\overset{A'}{\overset{\|}{C}}-(\overset{R_{19}}{\underset{R_{20}}{C}})_{l'}-(CH_2)_{m'}-(\overset{R_{21}}{\underset{R_{22}}{C}})_{n'}]_p-R_{23} \qquad (VI)$$

worin B, A′, $R_{19}$-$R_{23}$, l′, m′, n′ und p die genannten Bedeutungen haben, umsetzt

b) falls $R_{23}$ Halogen bedeutet, das erhaltene Kondensationsprodukt mit einer Verbindung der Formel $HNR_{24}R_{25}$, worin $R_{24}$ und $R_{25}$ die genannten Bedeutungen haben, umsetzt, und

c) falls $R_{23}$ eine geschützte Aminogruppe darstellt, die Schutzgruppe nach üblichen Methoden abspaltet.

2. Verfahren gemäß Anspruch 1, dadurch gekennzeichnet, daß in den Verbindungen der Formel I $R_1$ die OH-Gruppe und $R_{14}$ die Vinylgruppe bedeutet, einer der beiden Substituenten $R_6$ und $R_7$ OH, O-Alkyl oder O-Acyl bedeutet und der andere einen Rest der Formel

$$-O-\overset{O}{\overset{\|}{C}}-(\overset{R_{15}}{\underset{R_{16}}{C}})_{l}-[B-\overset{O}{\overset{\|}{C}}-(CH_2)_{m'}]_p-R_{23}$$

darstellt,

worin $R_{15}$, $R_{16}$, $R_{23}$, B, l und m′ die angegebene Bedeutung haben und p = 1 ist.

3. Verfahren gemäß Anspruch 2, dadurch gekennzeichnet, daß $R_{15}$ und $R_{16}$ jeweils Wasserstoff, B Sauerstoff, l die Zahl 1, m′ eine ganze Zahl von 1 bis 4 bedeuten und $R_{23}$ einen Rest der Formel $NR_{24}R_{25}$ darstellt, wobei $R_{24}$ und $R_{25}$ die genannten Bedeutungen haben.

4. Verfahren gemäß Anspruch 3, dadurch gekennzeichnet, daß einer der beiden Substituenten $R_6$ und $R_7$ die OH- oder Acetylgruppe bedeutet.

5. Arzneimittel, gekennzeichnet durch einen Gehalt an einer Verbindung gemäß Anspruch 1 und pharmazeutisch unbedenklichen Hilfs- und/oder Trägerstoffen.

6. Verwendung einer Verbindung gemäß Anspruch 1 zur Herstellung eines Arzneimittels mit positiv inotropen und/oder blutdrucksenkender Wirkung.

7. Verwendung einer Verbindung gemäß Anspruch 1 zur Herstellung eines Arzneimittels mit Augeninnendruck-senkender Wirkung.